# EUROPEAN PATENT APPLICATION

(11) **EP 4 556 006 A1**
(43) Date of publication of application: **21.05.2025**
(21) Application number: 23839693.1
(22) Date of filing: 14.07.2023
(51) Int. Cl.: A61K 31/423, A61P 3/06

(54) **DRUG FOR LOWERING BLOOD LDL CHOLESTEROL**

(30) Priority: 15.07.2022 JP 2022113930
(71) Applicant: KOWA COMPANY, LTD., Naka-ku Nagoya-shi Aichi 460-8625 (JP)
(72) Inventor: TANIGAWA, Ryohei, Tokyo 103-8433 (JP)
(74) Representative: Wächtershäuser & Hartz Patentanwaltspartnerschaft mbB
(86) International application number: PCT/JP2023/025980
(87) International publication number: WO 2024/014524

(57) **Abstract**

A novel preventive and/or therapeutic agent useful in the prevention and/or treatment of a disease on which a therapeutic effect can be expected by a reduction in blood LDL-C. The present invention relates to a medicine for preventing and/or treating a disease on which a therapeutic effect can be expected by a reduction in blood LDL-C, the medicine including a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.

## Description

### Field of the Invention

The present invention relates to an agent for reducing blood LDL cholesterol.

### Background of the Invention

Many epidemiological investigations have shown that the onset rate and mortality rate of a coronary artery disease increase along with an increase in blood LDL cholesterol (LDL-C). In recent years, it has been successively reported that an active reduction in blood LDL-C suppresses the onset of a cardiovascular event in a patient having a risk of the onset of the coronary artery disease. Accordingly, a therapy for reducing the blood LDL-C has been regarded as being clinically important. In view of such background, in medical guidelines in various countries, a target for the management of the blood LDL-C has been set in accordance with the risk of the onset of the coronary artery disease (Non-Patent Documents 1 to 3).

Improvements in lifestyle habits including a diet therapy and an exercise therapy are fundamental to the management of the blood LDL-C. However, a drug therapy is required for a patient in whom it is difficult to manage the blood LDL-C only through the improvements in lifestyle habits. In the drug therapy, an HMG-CoA reductase inhibitor (hereinafter referred to as "statin") has been recommended as a first-choice drug. However, in many patients, target values for blood LDL-C management may not be achieved only with statin, and hence addition of a therapeutic drug except statin, such as ezetimibe or a PCSK9 inhibitor, is required for these patients (Non-Patent Documents 1 to 3). In addition, there exist a certain number of patients in whom administration of statin is not to be continued owing to side effects, such as a muscle-related side effect and liver damage, and hence a therapeutic drug except the statin, such as ezetimibe or the PCSK9 inhibitor, is also required for these patients (Non-Patent Documents 4 and 5). However, the alternatives of drug therapies intended for reduction in blood LDL-C are limited, and hence a novel therapeutic drug may be useful in sufficiently managing the blood LDL-C.

Meanwhile, Patent Document 1 discloses that a compound of the following formula (1) or a salt thereof, or a solvate thereof has a selective PPARα-activating effect, and is hence useful as a preventive and/or therapeutic agent for diabetes, a diabetic complication (e.g., diabetic nephropathy), inflammation, a heart disease, or the like that is not accompanied by a weight gain or obesity in a mammal including a human: 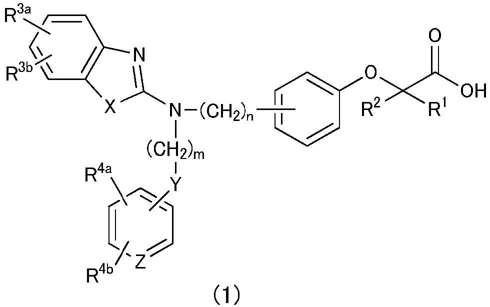 where R¹ and R² may be identical to or different from each other, and each represents a hydrogen atom, a methyl group, or an ethyl group, R^{3a}, R^{3b}, R^{4a}, and R^{4b} may be identical to or different from each other, and each represents a hydrogen atom, a halogen atom, a nitro group, a hydroxy group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, or R^{3a} and R^{3b}, or R^{4a} and R^{4b} are bonded to each other to represent an alkylenedioxy group, X represents an oxygen atom, a sulfur atom, or N-R⁵ (R⁵ represents a hydrogen atom, a C₁₋₄ alkyl group, a C₁₋₄ alkylsulfonyl group, or a C₁₋₄ alkyloxycarbonyl group), Y represents an oxygen atom, a S(O)₁ group ("l" represents a number of from 0 to 2), a carbonyl group, a carbonylamino group, an aminocarbonyl group, a sulfonylamino group, an aminosulfonyl group, or an NH group, Z represents CH or N, **"n"** represents a number of from 1 to 6, and **"m"** represents a number of from 2 to 6.

However, there is no description or suggestion of what effect such compound has on blood LDL-C.

### Prior Art Documents

### Patent Document

[Patent Document 1] WO 2005/023777 A1

### Non-Patent Documents

[Non-Patent Document 1] Japan Atherosclerosis Society (edition): Japan Atherosclerosis Society (JAS) Guidelines for Prevention of Atherosclerotic Cardiovascular Diseases 2017, Japan Atherosclerosis Society, 2017
[Non-Patent Document 2] Grundy SM, Stone NJ, Bailey AL, et al. 2018 AHA/ACC/AACVPR/AAPA/ABC/ACPM/ADA/AGS/APhA/ASPC/NLA/PCNA Guideline on the Management of Blood Cholesterol: A Report of the American College of Cardiology/American Heart Association Task Force on Clinical Practice Guidelines. Circulation. 2019 Jun 18; 139(25): e1082-e1143
[Non-Patent Document 3] Authors/Task Force Members 2019 ESC/EAS guidelines for the management of dyslipidaemias: Lipid modification to reduce cardiovascular risk. Atherosclerosis 2019 Nov; 290: 140-205
[Non-Patent Document 4] Rodrigo Alonso1, Ada Cuevas1, Alberto Cafferata. Diagnosis and Management of Statin Intolerance. J Atheroscler Thromb, 2019; 26: 207-215
[Non-Patent Document 5] Statin Intolerance Clinical Guide Working Group: Kouji Kajinami, Kazuhisa Tsukamoto, Shinji Koba, et al., Statin Intolerance Clinical Guide 2018

### Summary of the Invention

An object of the present invention is to provide a preventive and/or therapeutic agent for a disease on which a therapeutic effect can be expected by reduction in blood LDL-C as well as to provide a novel agent for reducing blood LDL-C.

To achieve the above-mentioned object, the present inventors made energetic investigations, and as a result, found that to their complete surprise, a compound disclosed as Example 85 in Patent Document 1 described above, that is, (R)-2-[3-[[N-(benzoxazol-2-yl)-N-3-(4-methoxyphenoxy)propyl]aminomethyl]phenoxy]butyric acid (hereinafter sometimes referred to as "compound A" or "pemafibrate") reduced a blood LDL-C concentration, and was hence useful in the prevention and/or treatment of a disease on which a therapeutic effect was expected by reduction in blood LDL-C. In this way, the inventors completed the present invention.

That is, the present invention provides the following [1] to [28].
[1] An agent for reducing blood LDL cholesterol, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.
[2] The agent for reducing blood LDL cholesterol according to [1], wherein the agent for reducing blood LDL cholesterol is intended for administration to a patient in whom a reduction in blood LDL cholesterol with a statin is insufficient or a patient in whom administration of the statin is restricted.
[3] An agent for preventing and/or treating hypercholesterolemia, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.
[4] An agent for preventing and/or treating hyper-LDL-cholesterolemia, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.
[5] The agent according to [3] or [4], which is intended for administration to a patient in whom a reduction in blood LDL cholesterol with a statin is insufficient or a patient in whom administration of the statin is restricted.
[6] A pharmaceutical composition for preventing and/or treating hypercholesterolemia, comprising a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.
[7] A pharmaceutical composition for preventing and/or treating hyper-LDL-cholesterolemia, comprising a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.
[8] The pharmaceutical composition according to [6] or [7], wherein the pharmaceutical composition is intended for administration to a patient in whom a reduction in blood LDL cholesterol with a statin is insufficient or a patient in whom administration of the statin is restricted.
[9] A method of reducing blood LDL cholesterol, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.
[10] The method according to [9], wherein the patient is a patient in whom a reduction in blood LDL cholesterol with a statin is insufficient or a patient in whom administration of the statin is restricted.
[11] A method of preventing and/or treating hypercholesterolemia, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.
[12] A method of preventing and/or treating hyper-LDL-cholesterolemia, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.
[13] The method according to [11] or [12], wherein the patient is a patient in whom a reduction in blood LDL cholesterol with a statin is insufficient or a patient in whom administration of the statin is restricted.
[14] Use of (R)-2-[3-[[N-(benzoxazol-2-yl)-N-3-(4-methoxyphenoxy)propyl]aminomethyl]phenoxy]butyric acid or a salt thereof, or a solvate thereof for reducing blood LDL cholesterol.
[15] The use according to [14], wherein the (R)-2-[3-[[N-(benzoxazol-2-yl)-N-3-(4-methoxyphenoxy)propyl]aminomethyl]phenoxy]butyric acid or a salt thereof, or a solvate thereof is administered to a patient in whom a reduction in blood LDL cholesterol with a statin is insufficient or a patient in whom administration of the statin is restricted.
[16] Use of (R)-2-[3-[[N-(benzoxazol-2-yl)-N-3-(4-methoxyphenoxy)propyl]aminomethyl]phenoxy]butyric acid or a salt thereof, or a solvate thereof for preventing and/or treating hypercholesterolemia.
[17] Use of (R)-2-[3-[[N-(benzoxazol-2-yl)-N-3-(4-methoxyphenoxy)propyl]aminomethyl]phenoxy]butyric acid or a salt thereof, or a solvate thereof for preventing and/or treating hyper-LDL-cholesterolemia.
[18] The use according to [16] or [17], wherein the (R)-2-[3-[[N-(benzoxazol-2-yl)-N-3-(4-methoxyphenoxy)propyl]aminomethyl]phenoxy]butyric acid or a salt thereof, or a solvate thereof is administered to a patient in whom a reduction in blood LDL cholesterol with a statin is insufficient or a patient in whom administration of the statin is restricted.
[19] Use of (R)-2-[3-[[N-(benzoxazol-2-yl)-N-3-(4-methoxyphenoxy)propyl]aminomethyl]phenoxy]butyric acid or a salt thereof, or a solvate thereof for production of an agent for reducing blood LDL cholesterol.
[20] The use according to [19], wherein the agent for reducing blood LDL cholesterol is an agent for reducing blood LDL cholesterol for administration to a patient in whom a reduction in blood LDL cholesterol with a statin is insufficient or a patient in whom administration of the statin is restricted.
[21] Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for preventing and/or treating hypercholesterolemia.
[22] Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for preventing and/or treating hyper-LDL-cholesterolemia.
[23] The use according to [21] or [22], wherein the pharmaceutical composition is a pharmaceutical composition for administration to a patient in whom a reduction in blood LDL cholesterol with a statin is insufficient or a patient in whom administration of the statin is restricted.
[24] (R)-2-[3-[[N-(Benzoxazol-2-yl)-N-3-(4-methoxyphenoxy)propyl]aminomethyl]phenoxy]butyric acid or a salt thereof, or a solvate thereof for use in reduction of blood LDL cholesterol.
[25] The (R)-2-[3-[[N-(benzoxazol-2-yl)-N-3-(4-methoxyphenoxy)propyl]aminomethyl]phenoxy]butyric acid or a salt thereof, or a solvate thereof according to [24], wherein the (R)-2-[3-[[N-(benzoxazol-2-yl)-N-3-(4-methoxyphenoxy)propyl]aminomethyl]phenoxy]butyric acid or a salt thereof, or a solvate thereof is intended for administration to a patient in whom a reduction in blood LDL cholesterol with a statin is insufficient or a patient in whom administration of the statin is restricted.
[26] (R)-2-[3-[[N-(Benzoxazol-2-yl)-N-3-(4-methoxyphenoxy)propyl]aminomethyl]phenoxy]butyric acid or a salt thereof, or a solvate thereof for use in prevention and/or treatment of hypercholesterolemia.
[27] (R)-2-[3-[[N-(Benzoxazol-2-yl)-N-3-(4-methoxyphenoxy)propyl]aminomethyl]phenoxy]butyric acid or a salt thereof, or a solvate thereof for use in prevention and/or treatment of hyper-LDL-cholesterolemia.
[28] The (R)-2-[3-[[N-(benzoxazol-2-yl)-N-3-(4-methoxyphenoxy)propyl]aminomethyl]phenoxy]butyric acid or a salt thereof, or a solvate thereof according to [26] or [27], wherein the (R)-2-[3-[[N-(benzoxazol-2-yl)-N-3-(4-methoxyphenoxy)propyl]aminomethyl]phenoxy]butyric acid or a salt thereof, or a solvate thereof is intended for administration to a patient in whom a reduction in blood LDL cholesterol with a statin is insufficient or a patient in whom administration of the statin is restricted.

### Advantageous Effects of Invention

The present invention provides a novel drug useful in the prevention and/or treatment of a disease on which a therapeutic effect can be expected by a reduction in blood LDL-C. According to the present invention, it is possible to provide a novel agent for reducing blood LDL-C to a patient in whom a reduction in blood LDL-C with a statin is insufficient or a patient in whom the administration of the statin is restricted, and a novel preventive and/or therapeutic alternative to a patient of the disease on which the therapeutic effect can be expected by the reduction in blood LDL-C.

### Brief Description of Drawings

FIG. 1 shows the rate of a change in fasting blood LDL-C concentration at the time of the administration of the compound A (0.4 mg per day) to a patient of a nonalcoholic fatty liver disease.
FIG. 2 shows the rate of a change in fasting blood LDL-C concentration at the time of the administration of the compound A (0.4 mg per day) to the patient of the nonalcoholic fatty liver disease.
FIG. 3 shows the rates of changes from the baselines of a fasting blood lathosterol concentration, a fasting blood sitosterol concentration, and a fasting blood campesterol concentration at the time of the administration of the compound A (0.4 mg per day) to the patient of the nonalcoholic fatty liver disease.

### Embodiments for Carrying out the Invention

(R)-2-[3-[[N-(Benzoxazol-2-yl)-N-3-(4-methoxyphenoxy)propyl]aminomethyl]phenoxy]butyric acid (compound A) to be used in the present invention is represented by the following chemical formula (A):

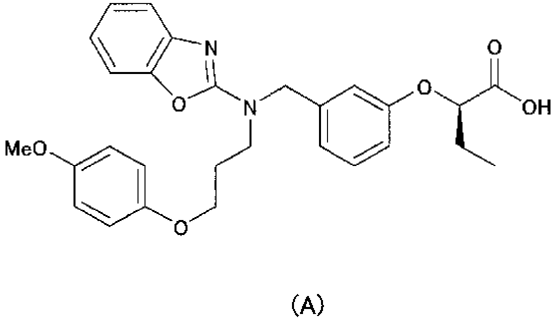

The compound A may be produced in accordance with, for example, a method described in WO 2005/023777 A1. The compound A may be formulated into a preparation in accordance with a method described in an academic literature. A preparation containing the compound A is approved as a therapeutic agent for hyperlipidemia "PARMODIA (registered trademark) TABLET" in Japan, and the "PARMODIA TABLET" may be used.

In one embodiment of the present invention, a salt or a solvate of the compound A may be used. The salt and the solvate may each be produced by an ordinary method. While the salt of the compound A is not particularly limited as long as the salt is pharmaceutically acceptable, examples thereof include: alkali metal salts, such as a sodium salt and a potassium salt; alkaline earth metal salts, such as a calcium salt and a magnesium salt; organic base salts, such as an ammonium salt and a trialkylamine salt; mineral acid salts, such as a hydrochloride and a sulfate; and organic acid salts such as an acetate. Examples of the solvate of the compound A or of the salt thereof include a hydrate and an alcohol adduct **(e.g.,** an ethanol adduct).

As described in Examples described later, it has been shown that the compound A reduces a blood LDL-C concentration, and reduces both a synthesis marker and an absorption marker for cholesterol. It is conceivable from the foregoing that the compound A has the following effect: the compound suppresses the synthesis of LDL-C in a body and the absorption of cholesterol from a diet to reduce blood LDL-C. Accordingly, the compound A or a salt thereof, or a solvate thereof may serve as an active ingredient for an agent for reducing blood LDL-C. In addition, the compound A or a salt thereof, or a solvate thereof is useful in the prevention and/or treatment of a disease on which a therapeutic effect can be expected by a reduction in blood LDL-C through the reduction in blood LDL-C concentration.

Unless otherwise stated, the term "agent for reducing blood LDL-C" as used herein refers to, for example, a drug for a disease or the like on which a therapeutic effect can be expected by a reduction in blood LDL-C through its application. Specifically, the term refers to, for example, a drug for a disease or the like on which a therapeutic effect can be expected by a reduction in blood LDL-C through the inhibition of the synthesis of LDL-C in a living organism or the absorption of cholesterol therein. The mode of the reduction in LDL-C is not particularly limited, and examples thereof include: the suppression of the synthesis of the LDL-C; the inhibition of the absorption of the cholesterol; and the promotion of the excretion of the cholesterol.

The term "disease on which a therapeutic effect can be expected by a reduction in blood LDL-C" as used herein refers to, for example, hypercholesterolemia, familial hypercholesterolemia (homozygote), or familial hypercholesterolemia (heterozygote). Of those, hypercholesterolemia is preferred in the present invention, and hyper-LDL-cholesterolemia is more preferred.

The patients of those diseases include a patient on whom an existing agent for reducing LDL-C exhibits an insufficient effect such as a patient in whom a reduction in blood LDL-C with a statin is insufficient. In addition, the patients include a patient for whom treatment with the existing agent for reducing LDL-C is unsuitable such as a patient in whom the administration of the statin is restricted. Herein, the term "patient in whom a reduction in blood LDL-C with a statin is insufficient" refers to a patient in whom a reduction in blood LDL-C is not sufficiently achieved even under the administration of the statin. In addition, the term "patient in whom the administration of the statin is restricted" refers to a patient in whom the administration of the statin is restricted by contraindications, such as a muscle-related side effect and liver damage.

In the present invention, an administration subject is not particularly limited. However, from the viewpoint of providing a novel alternative of a drug therapy intended for a reduction in blood LDL-C, a patient in whom a reduction in blood LDL cholesterol with a statin is insufficient or a patient in whom the administration of the statin is restricted is preferred.

In the present invention, the compound A or a salt thereof, or a solvate thereof may be used alone because the compound A or a salt thereof, or a solvate thereof itself reduces a blood LDL-C concentration. However, in one aspect of the present invention, from the viewpoint of reducing blood LDL-C, the compound A or a salt thereof, or a solvate thereof may be used in combination with any other agent for reducing LDL-C intended for a reduction in blood LDL-C. Examples of such drug include an anion exchange resin, a statin, a small intestine cholesterol transporter inhibitor, and a PCSK9 inhibitor.

Examples of the anion exchange resin include colestyramine and colestimide. With regard to colestyramine, for example, QUESTRAN (registered trademark) powder 44.4% is available as a commercial drug. With regard to colestimide, for example, CHOLEBINE (registered trademark) tablet 500 mg is available as a commercial drug.

The statin is, for example, pravastatin, simvastatin, fluvastatin, atorvastatin, pitavastatin, rosuvastatin, or a salt thereof, or a solvate thereof.

The small intestine cholesterol transporter inhibitor is, for example, ezetimibe. With regard to ezetimibe, for example, ZETIA (registered trademark) tablet 10 mg is available as a commercial drug.

The PCSK9 inhibitor is, for example, a human anti-PCSK-9 monoclonal antibody preparation. With regard to the human anti-PCSK-9 monoclonal antibody preparation, for example, REPATHA (registered trademark) subcutaneous injection 140 mg pen is available as a commercial drug.

In one aspect of the present invention, the compound A or a salt thereof, or a solvate thereof, when used as a medicine, may be formulated into a preparation, such as a tablet, a capsule, a granule, a powder, a lotion, an ointment, an injection, or a suppository, through use of any other pharmaceutically acceptable carrier as required. Those preparations may each be produced by a known method. Examples of the pharmaceutically acceptable carrier may include an excipient, a disintegrator, a binder, a lubricant, a plasticizer, a glidant, a diluent, a solubilizer, a suspending agent, an isotonizing agent, a pH adjuster, a buffer, a stabilizer, a coating agent, a colorant, a taste-masking agent, and an odor-masking agent.

In one aspect of the present invention, the compound A or a salt thereof, or a solvate thereof may be administered by oral administration or parenteral administration, and oral administration is preferred. The therapeutically effective dose of the compound A or the salt thereof, or the solvate thereof, and the number of times of the administration thereof may be appropriately set by a person skilled in the art, though the dose and the number vary depending on, for example, the body weight, age, sex, and symptom of a patient. For example, for a normal adult, the compound A may be administered in a dose of from 0.05 mg to 0.8 mg per day in 1 to 3 portions, and is administered preferably in a dose of from 0.1 mg to 0.4 mg per day in 1 or 2 portions, more preferably in a dose of from 0.2 mg to 0.4 mg per day in 1 or 2 portions.

### Examples

The present invention is more specifically described below by way of Examples. However, these Examples are not intended to limit the present invention.

### Example 1: Investigation on Effect of Compound A on Blood LDL-C

An effect on blood LDL-C at the time of the oral administration of 0.4 mg per day of the compound A (a **0.2-**milligram tablet of the compound A was administered twice a day at the time of fasting, 58 cases) to a patient of a nonalcoholic fatty liver disease (NAFLD) for 12 weeks was investigated by comparison to the administration of a placebo (60 cases).

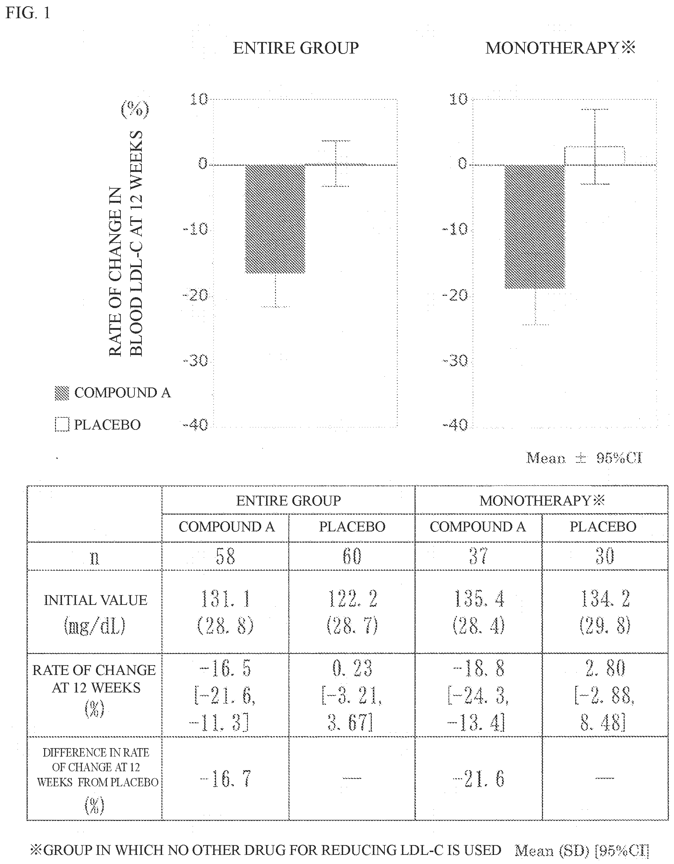

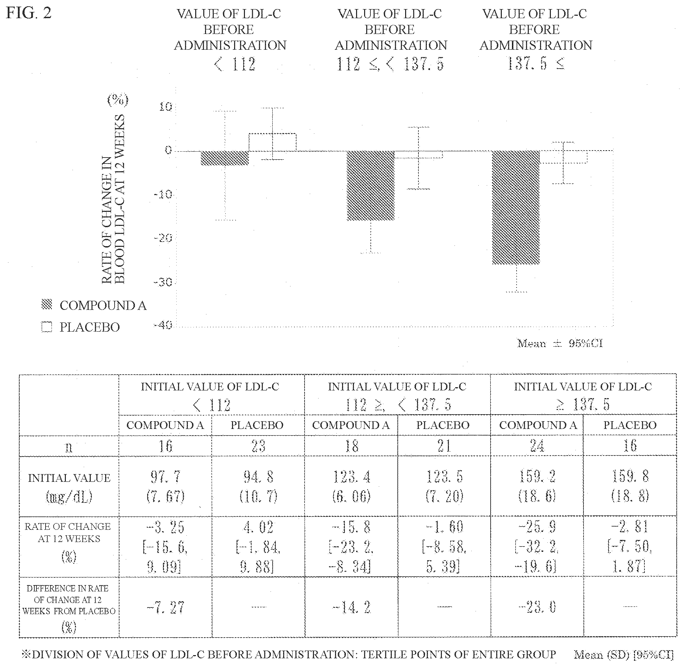

FIG. 1 shows the rate (%) of a change from the baseline of a blood LDL-C concentration at the time point of 12 weeks after the start of the administration. FIG. 2 shows the rate (%) of a change for each initial value of the blood LDL-C.

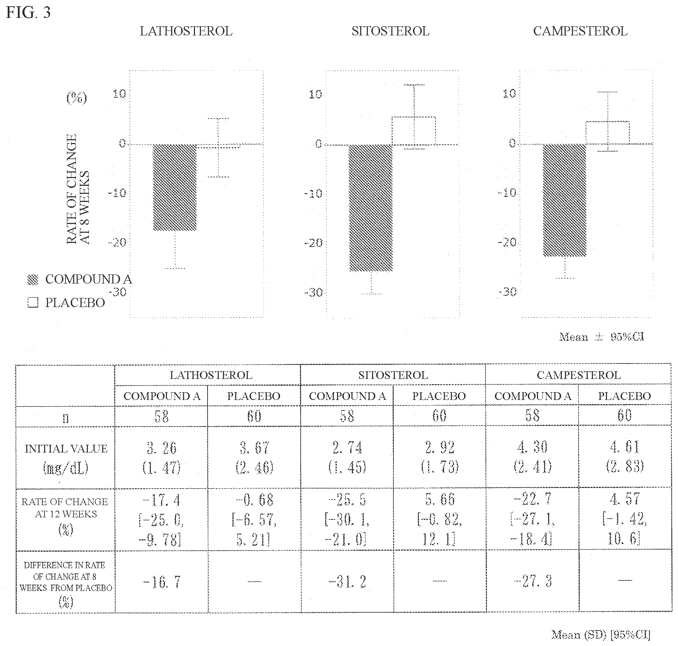

FIG. 3 shows the rate (%) of a change from the baseline of a blood lathosterol concentration, the rate (%) of a change from the baseline of a blood sitosterol concentration, and the rate (%) of a change from the baseline of a blood campesterol concentration at the time point of 8 weeks after the start of the administration.

It was made clear from FIG. 1 that in both an entire group including patients taking an existing agent for reducing LDL-C and patients who did not take any existing agent for reducing LDL-C, the administration of the compound A reduced a fasting blood LDL-C concentration as compared to the placebo administration. It was also made clear from FIG. 2 that the reducing effect of the compound A on LDL-C became larger as the blood LDL-C concentration before the administration thereof increased.

It was made clear from FIG. 3 that the administration of the compound A reduced the fasting blood concentration of lathosterol serving as a synthesis marker for cholesterol as compared to the placebo administration. It was also made clear that the administration of the compound A reduced the fasting blood concentration of sitosterol and the fasting blood concentration of campesterol, the sterols each serving as an absorption marker for cholesterol, as compared to the placebo administration.

### Industrial Applicability

The present invention was completed on the basis of the fact that the inventors were the first to find the following, and the present invention is useful as a pharmaceutical drug for preventing and/or treating a disease on which a therapeutic effect can be expected by a reduction in blood LDL-C: the compound A has a clear reducing effect on a fasting blood LDL-C concentration.

## Claims

1. An agent for reducing blood LDL cholesterol, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.

2. The agent for reducing blood LDL cholesterol according to claim 1, wherein the agent for reducing blood LDL cholesterol is intended for administration to a patient in whom a reduction in blood LDL cholesterol with a statin is insufficient or a patient in whom administration of the statin is restricted.

3. An agent for preventing and/or treating hypercholesterolemia, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.

4. An agent for preventing and/or treating hyper-LDL-cholesterolemia, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.

5. The agent according to claim 3 or 4, wherein the preventive and/or therapeutic agent is intended for administration to a patient in whom a reduction in blood LDL cholesterol with a statin is insufficient or a patient in whom administration of the statin is restricted.

6. A method of reducing blood LDL cholesterol, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.

7. The method according to claim 6, wherein the patient is a patient in whom a reduction in blood LDL cholesterol with a statin is insufficient or a patient in whom administration of the statin is restricted.

8. A method of preventing and/or treating hypercholesterolemia, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.

9. A method of preventing and/or treating hyper-LDL-cholesterolemia, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.

10. The method according to claim 8 or 9, wherein the patient is a patient in whom a reduction in blood LDL cholesterol with a statin is insufficient or a patient in whom administration of the statin is restricted.

11. Use of (R)-2-[3-[[N-(benzoxazol-2-yl)-N-3-(4-methoxyphenoxy)propyl]aminomethyl]phenoxy]butyric acid or a salt thereof, or a solvate thereof for production of an agent for reducing blood LDL cholesterol.

12. The use according to claim 11, wherein the agent for reducing blood LDL cholesterol is an agent for reducing blood LDL cholesterol for administration to a patient in whom a reduction in blood LDL cholesterol with a statin is insufficient or a patient in whom administration of the statin is restricted.

13. Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for preventing and/or treating hypercholesterolemia.

14. Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for preventing and/or treating hyper-LDL-cholesterolemia.

15. The use according to claim 13 or 14, wherein the pharmaceutical composition is a pharmaceutical composition for administration to a patient in whom a reduction in blood LDL cholesterol with a statin is insufficient or a patient in whom administration of the statin is restricted.

16. (R)-2-[3-[[N-(Benzoxazol-2-yl)-N-3-(4-methoxyphenoxy)propyl]aminomethyl]phenoxy]butyric acid or a salt thereof, or a solvate thereof for use in reduction of blood LDL cholesterol.

17. The (R)-2-[3-[[N-(benzoxazol-2-yl)-N-3-(4-methoxyphenoxy)propyl]aminomethyl]phenoxy]butyric acid or a salt thereof, or a solvate thereof according to claim 16, wherein the (R)-2-[3-[[N-(benzoxazol-2-yl)-N-3-(4-methoxyphenoxy)propyl]aminomethyl]phenoxy]butyric acid or a salt thereof, or a solvate thereof is intended for administration to a patient in whom a reduction in blood LDL cholesterol with a statin is insufficient or a patient in whom administration of the statin is restricted.

18. (R)-2-[3-[[N-(Benzoxazol-2-yl)-N-3-(4-methoxyphenoxy)propyl]aminomethyl]phenoxy]butyric acid or a salt thereof, or a solvate thereof for use in prevention and/or treatment of hypercholesterolemia.

19. (R)-2-[3-[[N-(Benzoxazol-2-yl)-N-3-(4-methoxyphenoxy)propyl]aminomethyl]phenoxy]butyric acid or a salt thereof, or a solvate thereof for use in prevention and/or treatment of hyper-LDL-cholesterolemia.

20. The (R)-2-[3-[[N-(benzoxazol-2-yl)-N-3-(4-methoxyphenoxy)propyl]aminomethyl]phenoxy]butyric acid or a salt thereof, or a solvate thereof according to claim 18 or 19, wherein the (R)-2-[3-[[N-(benzoxazol-2-yl)-N-3-(4-methoxyphenoxy)propyl]aminomethyl]phenoxy]butyric acid or a salt thereof, or a solvate thereof is intended for administration to a patient in whom a reduction in blood LDL cholesterol with a statin is insufficient or a patient in whom administration of the statin is restricted.
